# EUROPEAN PATENT SPECIFICATION

(11) **EP 1 912 619 B1**
(45) Date of publication and mention of the grant of the patent: **19.11.2008**
(21) Application number: 06776432.4
(22) Date of filing: 25.07.2006
(51) Int. Cl.: A61K 8/26, A61K 8/34, A61K 8/37, A61K 8/39, A61Q 15/00

(54) **ANTIPERSPIRANT COMPOSITIONS**
ANTIPERSPIRANT-ZUSAMMENSETZUNGEN
COMPOSITIONS ANTITRANSPIRATION

(30) Priority: 10.08.2005 GB 0516418
(43) Date of publication of application: 23.04.2008
(73) Proprietor: Unilever PLC, London Greater London EC4P 4BQ (GB); Unilever N.V., 3013 AL Rotterdam (NL)
(72) Inventor: FRANKLIN, Kevin, Ronald, Bebington, Wirral Merseyside CH63 3JW (GB); STOCKTON, Joanne Elisabeth, Bebington, Wirral, Merseyside CH63 3JW (GB); KENCH, Joyce, Bebington, Wirral, Merseyside CH63 3JW (GB)
(74) Representative: Whaley, Christopher
(86) International application number: PCT/EP2006/007395
(87) International publication number: WO 2007/017119

(56) References cited:
- EP-A1- 0 272 354
- WO-A-91/18587
- US-A- 4 065 564
- US-A- 5 989 531

## Description

The present invention relates to antiperspirant (AP) compositions suitable for use in treatment of the human body. The invention further relates to methods for preparing such compositions, and products employing such compositions.

AP compositions have been known for many years and a wide variety of such compositions have been marketed commercially. Some of the known AP compositions have used solubilised AP active and give some of the benefits of the present invention. The most common of such compositions use water to aid the solubilisation of the AP active; however, aqueous compositions suffer from many disadvantages, including those described hereinafter.

Anhydrous AP compositions employing solubilised AP active have not been widely marketed, particularly in compositions used as a liquid products; however such compositions have been disclosed in the patent literature. Many such disclosures are of compositions comprising alcohol-soluble aluminium compounds that are complexed with propylene glycol, as described in US 3,359,169 (Revlon Inc., 1967). A particular example is US 4,065,564 (Lever Bros. Co., 1977), which describes anhydrous AP compositions comprising an aluminium chlorhydroxide complex dissolved in alcohol and small amounts of silicone oil and optional skin lubricants, such as isopropyl myristate. A related example is US 4,053,581 (Lever Bros. Co., 1977). More recently, there have been publications disclosing solutions of AP active in 1,2-diols such as 1,2-pentanediol and 1,2-hexanediol; WO 01/13869 (Procter & Gamble Co., 2001) and WO 02/094327 (Procter & Gamble Co., 2002) are examples of such publications.

One of the problems of AP compositions comprising a solution of an AP active in a short chain (C2-C6) alcohol, such as ethanol, is that such compositions may be perceived by some people as having negative sensory attributes, notably a tendency to irritate the skin. Another problem is that short chain (C2-C6) alcohols such as ethanol are classed as "VOC"s (volatile organic compounds) and that release into the atmosphere of VOCs is believed to have negative environmental consequences. This latter problem may be accentuated when the composition also comprises a volatile propellant.

It is an object of the present invention to provide an AP composition comprising a solubilised AP active that is not perceived by consumers as having negative sensory attributes, in particular not cause irritation.

It is a further object of the invention to provide an AP composition comprising a solubilised AP active that releases less ethanol into the atmosphere than AP compositions comprising a solubilised AP active as disclosed in the prior art.

It is a further object of the invention to provide an AP composition comprising a solubilised AP active that has good stability, particularly at high and low temperatures.

It is a further object of the invention to provide an AP composition comprising a solubilised AP active that delivers good antiperspirancy performance.

According to a first aspect of the invention, there is provided an anhydrous antiperspirant composition comprising from 2 to 25% by weight of solubilised aluminium-containing antiperspirant active; from 20 to 50% by weight of ethanol and from 20 to 55% by weight of one or more solubiliser oils selected from the group consisting of:
(i). a branched-chain fatty alcohol having from 16 to 24 carbon atoms;
(ii). a benzoate ester of a short chain (C2-C6) alcohol substituted with a phenyl, phenoxy or benzoyloxy group;
(iii). a benzoate ester of a straight-chain alcohol of from 6 to 8 carbon atoms; and
(iv). a propoxylated linear fatty alcohol having a degree of propoxylation of from 2 to 4 and a fatty alcohol chain length of from 10 to 18 carbon atoms.
wherein the ratio of antiperspirant active to ethanol and of antiperspirant active to the solubiliser oil is from 1:10 to 1:1 by weight and the ratio of ethanol to oil is from 1:2 to 2:1 by weight.

According to a second aspect of the invention, there is provided a cosmetic method of reducing perspiration comprising the application to the surface of the human body of a composition according to the first aspect of the present invention.

According to a third aspect of the invention, there is provided a method of manufacture of a composition according to the first aspect of the present invention.

According to a fourth aspect of the invention, there is provided a product comprising an anhydrous antiperspirant composition according to the first aspect of the present invention and a spray dispenser, roll-on dispenser, or porous head dispenser.

The AP compositions of the invention are suitable for use as cosmetic compositions in the cosmetic treatment of the human body. Their principle function is to reduce perspiration, particularly in the areas of the body where this is most required, notably the underarm regions and the feet.

Compositions according to the invention have the feature that the AP active is solubilised. This feature may deliver sensory benefits in that the composition may feel less gritty than compositions comprising particulate AP active. Alternatively or additionally, there may also be delivery benefits in that solubilised AP active is less likely to lead to blockage of the product applicator. Examples of applicators that are prone to such blockage include roll-on applicators, the roll-on sometimes becoming difficult to move; spray devices, the narrow apertures in the nozzle sometimes becoming blocked, and porous head applicators, the pores sometimes becoming blocked.

The compositions of the invention are anhydrous, meaning that they comprise less than 10% by weight of water. Preferably they comprise less than 5%, more preferably less than 1%, and most preferably less than 0.5% by weight of water. Anhydrous compositions may have sensory benefits in terms of feeling drier than aqueous compositions. Another possible benefit is that they can be packaged in a metallic container with reduced risk of corrosion of the container. A further possible benefit is that they may deliver enhanced antiperspirancy performance.

In the compositions of the invention, all of the AP active is solubilised, meaning that it exist as a solution in the other components therein present. This solution may be gelled to form a stick or soft solid, it may be used as a liquid, or it may be diluted with a volatile propellant and used in an aerosol. The benefits of the invention are of particular relevance when the composition is a liquid. Thus, compositions according to the invention are preferably liquid, meaning that they are able to flow under gravity at ambient temperature.

Throughout this specification, the term "solution" should be understood to refer to a homogeneous, single phase mixture that is optically clear.

In all possible product forms, the composition may comprise additional insoluble material, typically uniformly dispersed throughout the product; however, in preferred compositions, particularly those used as a liquid product, the composition is itself a solution. In such solution compositions, the benefits of having solubilised AP active (*vide supra*) are of particular relevance.

Compositions according to the invention that are used as liquid products may be roll-on compositions, spray compositions, or compositions applied through a porous head dispenser. The compositions of the invention are particular suitable for use as spray compositions, especially spray compositions that do not comprise a volatile propellant, such as trigger spray and pump spray compositions.

The AP active is preferably soluble in ethanol, typically meaning that a 50% w/w solution of the active in ethanol is clear at ambient temperature. Throughout this specification, ambient temperature shall be understood to mean about 23°C. Examples of suitable AP actives are aluminium-containing astringent AP salts complexed with a polyhydric alcohol, such aluminium chlorohydrate (ACH) complexed with propylene glycol (PG). Particular ACH-PG actives that have been found suitable are Reach 301 PGO, Reach 301 PG, and Rehydrol II, all available from Reheis Inc.

The total amount of AP active in the compositions of the invention is from 2 to 25%, preferably from 5 to 22%, and more preferably from 10 to 20% by weight of the composition. A certain minimum amount of AP active is required in order to give an acceptable antiperspirancy performance. The maximum level of AP active is set because of the fall off of performance at high levels and in order to ease the formulation of stable compositions.

Ethanol is incorporated in the compositions of the invention at a level of from 20 to 50%, preferably from 25 to 45%, and more preferably from 30 to 40% by weight of the composition. The weight ratio of AP active to ethanol is from 1:10 to 1:1. The minimum weight ratio of AP active to ethanol is preferably from 1:5 and more preferably from 1:3. The maximum weight ratio of AP active to ethanol is preferably up to 2:3 and more preferably up to 1:2. A certain minimum amount of ethanol is required, particularly with respect to the AP salt, in order to aid the solubilsation of the AP active and give a stable composition. The maximum level of ethanol is set because some people perceive negative sensory attributes, notably irritancy, when ethanol is applied to the skin together with AP active. There is also a desire to keep the level of ethanol reasonably low in order to minimise its release into the atmosphere (*vide supra*).

The solubiliser oil serves to aid the solubilisation of the AP active and may also ameliorate the negative sensory attributes perceived by some people on application of ethanolic AP actives to their skin (*vide supra*). The inventors have noticed that only certain oils are able to sufficiently aid the solubilisation of the AP active and details of these oils are provided below.

The solubiliser oil is selected from the group of oils listed as (i) to (iv) in the first embodiment of the invention. In the following description, when a particular solubiliser oil, selection of solubilisation oils, or combination of solubilisation oils is described as "preferred", this is because said oil or oils enable the formulation of particularly stable compositions.

It is preferred that the solubiliser oil is selected from the group consisting of:
(i). a branched-chain fatty alcohol having from 16 to 24 carbon atoms; and
(ii). a benzoate ester of a short chain (C2-C6) alcohol substituted with a phenyl, phenoxy or benzoyloxy group.

It is more preferred that the solubiliser oil is a branched-chain fatty alcohol having from 16 to 24 carbon atoms. In such embodiments, an additional solubiliser oil that is a benzoate ester of a short chain (C2-C6) alcohol substituted with a benzoyloxy group may be present in order to add to the stability of the composition.

When a solubiliser oil that is a branched-chain fatty alcohol having from 16 to 24 carbon atoms is used, it is typically a monohydric primary alcohol. It is preferred that the fatty chain is saturated; it more preferred that the saturated fatty chain is only singularly branched, i.e. only one methine (tertiary) carbon atom is present and no quaternary carbon atoms are present. Particularly preferred solubiliser oils of this type are isostearyl alcohol (ISA), as sold by Uniqema under the trade name Prisorine 3515, and octyldocecanol, as sold by Cognis GmbH under the trade name Eutanol G. Of these particularly preferred solubiliser oils, ISA is most preferred.

When a solubiliser oil that is a benzoate ester of a short chain (C2-C6) alcohol substituted with a phenyl, phenoxy or benzoyloxy group is used, the short chain alcohol may be linear (i.e. straight chain) or branched chain and may optionally comprise ether linkages. Preferably, the short chain alcohol is linear or methyl-branched (i.e. having methyl substituents) and the solubiliser oil comprises no atoms other than carbon, hydrogen, and oxygen. Particular solubiliser oils of this type are di(propylene glycol) dibenzoate, as sold by Finetex Inc. under the trade name Finsolv PG22; phenylethyl benzoate, as sold by Finetex Inc. under the trade name Finsolv SUN; and 1-methyl-2-phenoxyethyl benzoate, available from Degussa AG as DG1. Particularly preferred solubiliser oils of this type are benzoate esters of a short chain (C2-C6) alcohol substituted with a benzoyloxy group. These latter oils may be named alternatively as dibenzoate esters of a short chain (C2-C6) dihydric alcohol. Of these particularly preferred solubiliser oils, di(propylene glycol) dibenzoate is most preferred.

When a solubiliser oil that is a benzoate ester of a linear (i.e. straight chain) alcohol of from 6 to 8 carbon atoms is used, the esterified alcohol is typically a monohydric primary alcohol. It is preferred that the alcohol has a saturated hydrocarbon chain. A particularly preferred solubiliser oil of this type is octyl benzoate, as sold by Finetex Inc. under the trade name Finsolv EB.

When a solubiliser oil that is a propoxylated linear (i.e. straight chain) fatty alcohol having a degree of propoxylation of from 2 to 4 and a fatty alcohol chain length of from 10 to 18 carbon atoms is used, the fatty alcohol is typically a monohydric primary alcohol. It is preferred that the alcohol has a saturated hydrocarbon chain. A particularly preferred solubiliser oil of this type is PPG myristyl ether, as sold by Croda Inc. under the trade name Promistryl PM3.

The total amount of solubiliser oil present in the compositions of the invention is from 20 to 55%, preferably from 25 to 50%, more preferably from 30 to 45%, and most preferably from 35 to 45% by weight of the composition. The weight ratio of AP active to solubiliser oil is from 1:10 to 1:1. The minimum weight ratio of AP active to solubiliser oil is preferably from 1:5 and more preferably from 1:3. The maximum weight ratio of AP active to solubiliser oil is preferably up to 2:3 and more preferably up to 1:2. A certain minimum amount of solubiliser oil is required, particularly with respect to the AP salt, in order to aid the solubilisation of the AP active and in order to ameliorate any negative sensory perception resulting from the presence of the AP active and the ethanol. The maximum level of solubiliser oil is set for reasons of composition stability and because some people perceive negative sensory attributes, notably greasiness, when the solubilser oil is applied to the skin at a high level.

The ratio of ethanol to solubiliser oil is from 1:2 to 2:1 and preferably from 2:3 to 3:2 by weight. It desirable to have the amounts of ethanol and solubiliser oil reasonably well balanced in order to achieve good stability of the composition and in order to minimise negative sensory attributes and any environmental impact that large scale marketing of the composition might have (*vide supra*).

All of the above references to "solubiliser oil" are to the total of all of the solubiliser oils selected from the specified group, said group consisting of solubiliser oils (i) to (iv) listed in the first embodiment of the invention, unless otherwise specified.

Many other components may be present in compositions of the invention, their selection often being dictated by the desired product form.

A preferred additional component is a silicone oil. Such oils can enhance the sensory perception of the composition on application to the skin. Silicone oils may be used at from 1 to 20% and preferably at from 5 to 15% by weight of the composition. Volatile silicone oils are preferred, materials being classed as "volatile" when they have a measurable vapour pressure at ambient temperature and atmospheric pressure. Suitable volatile silicone oils, or polyorganosiloxanes as they are otherwise known, may be cyclic or linear. Preferred silicone oils are cyclic polydimethylisoxanes containing from 3 to 9 silicon atoms, preferably from 3 to 7 silicone atoms, and more preferably from 4 to 5 silicon atoms. Such polydimethylisoxanes are generally known as cyclomethicones and the most preferred of these materials for use in the present invention is cylcopentasiloxane. Suitable linear silicone oils include polydimethylsiloxanes containing from about 3 to about 9 silicon atoms. Examples of commercially available silicone oils suitable for use in the present invention include Dow Corning silicone fluids 344, 345, 244, 245, 246, 556, and the 200 series; Union Carbide silicone fluids 7207 and 7158; and General Electric silicone SF1202.

A short chain (C2-C6) polyhydric alcohol may be included in compositions according to the invention. Such materials may aid the solubilisation of the AP active and they may enhance the sensory perception of the composition when it is applied to the skin, sometimes delivering reduced stickiness. However, the inventors have also noted that such materials can have a detrimental effect on composition stability and they must only be used with caution. When employed, they are typically used at from 1 to 10% of the composition. Suitable short chain (C2-C6) polyhydric alcohols include propylene glycol, di(propylene glycol), glycerol, pentane-1,2-diol, and hexane-1,2-diol. Short chain (C2-C6) dihydric alcohols are preferred, particularly propylene glycol.

Perfume or fragrance may be beneficially included in compositions of the invention. Suitable materials include conventional perfumes, such as perfume oils and also include so-called deo-perfumes, as described in EP 545,556 and other publications. Levels of incorporation are preferably up to 4%, particularly from 0.1% to 3%, and especially from 0.5% to 2% by weight of the composition.

In compositions according to the invention, it may be desirable to include one or more wash-off aids, often in a proportion of up to about 10% by weight, especially up to about 5% by weight and particularly from 0.5 to 3% by weight. Such wash off aids commonly comprise nonionic surfactants and especially nonionic surfactants which contain a polyalkylene oxide moiety, the residue of a fatty acid or fatty alcohol, and optionally the residue of an aliphatic polyhydric alcohol linking group. Although, the surfactants may comprise a single fatty residue, they preferably contain two residues. Preferably, the surfactant is an ester surfactant, and especially a diester surfactant. The polyalkylene oxide is often polyethylene oxide, or polypropylene oxide or mixed polyethylene oxide/propylene oxide, the polymer containing from 3 to 50 and especially from 5 to 20 alkylene oxide units. The fatty residue often derives from a fatty acid or alcohol containing from 12 to 24 carbons, which in many instances is linear, examples including 16, 18 or 22 linear carbons. Especially preferred wash-off aids herein comprise polyethylene oxide diesters of fatty alcohols containing 16 to 22 linear carbons, such as PEG-8 distearate.

An additional deodorant active, such as an organic antimicrobial agent may be included in compositions of the invention. The level of incorporation is typically from 0.01% to 3% by weight of the composition. Suitable deodorant actives may act as bactericides, examples including quaternary ammonium compounds; like cetyltrimethylammonium salts; chlorhexidine and salts thereof; and diglycerol monocaprate, diglycerol monolaurate, glycerol monolaurate, and similar materials, as described in "Deodorant Ingredient", by S.A.Makin and M.R.Lowry, in "Antiperspirants and Deodorants", Ed. K. Laden (1999, Marcel Dekker, New York). Other suitable deodorant actives are polyhexamethylene biguanide salts, such as Cosmocil CQ available from Zeneca PLC; 2',4,4'-trichloro,2-hydroxydiphenyl ether (triclosan); and 3,7,11-trimethyldodeca-2,6,10-trienol (farnesol).

In some embodiments of the invention it is desirable to include a structurant or thickening agent. Such components are typically used in stick or soft solid compositions and may be used at from 0.1 to 25% and particularly at from 1 to 15% by weight of the composition. It is highly desirable that the structurant or thickening agent is compatible with the solution of AP active and does not cause the AP active to come out of solution.

In embodiments of the invention that are aerosol compositions, a volatile propellant is a desired additional component. Volatile propellants, by which it meant liquefied gases having a boiling point of below 10°C, are widely used in aerosol compositions. They are not considered preferred components of the compositions of the invention, because they tend to be VOCs (*vide supra*). However, suitable volatile propellants may be present at from 15% to 58% by weight of the composition. The volatile propellant should be selected such that it is miscible with the solution of AP active, the aerosol composition comprising the AP solution and volatile propellant being itself a homogeneous single phase solution.

Further additional components that may also be included are colourants and preservatives at a conventional level, for example C₁-C₃ alkyl parabens.

The compositions of the invention may be prepared by any suitable means. In a preferred method of manufacture, a solution of the solubiliser oil in ethanol is first prepared, typically at a concentration of from 40 to 70% by weight. Independently, a solution of the AP active in ethanol is prepared, typically at a concentration of from 40 to 60% and preferably at a concentration of about 50% by weight. The oil solution and AP active solution are then mixed, typically at a ratio of from 1:3 to 3:1, optionally with gentle agitation to produce the final composition. When other components, such as silicone oil or polyhydric alcohol, are to be included in the composition, these are preferably dissolved in the ethanolic oil solution before it is mixed with the AP active solution.

The most preferred compositions according to the invention are liquids and these may conveniently be applied to the human body using a spray dispenser, roll-on dispenser, or porous head dispenser, such as described in WO 04/062423 (Unilever, 2004).

In particularly preferred embodiments of the invention, a liquid composition according to the invention is applied to the human body using a spray dispenser. In such embodiments, it is especially preferred that the composition lacks volatile propellant, the composition being applied using a device that can generate a spray from such a composition. Suitable dispensers include pump sprays, squeeze sprays, trigger sprays, and other devices that are able to pressurise the liquid composition and force it through a nozzle.

A preferred feature of dispensers used in conjunction with liquid compositions according to the invention is that the liquid composition is held in a reservoir comprising a translucent or transparent wall or window that permits a user to recognise that the liquid composition is a clear solution. It is particularly desirable that opposed walls of the reservoir are translucent or transparent, thereby enabling a user to look though the dispenser and its contents.

### Examples

All amounts indicated in the tables are percentages by weight. Percentage figures referred to in the general text are also by weight.
A 50% solution of Reach 301 PGO antiperspirant complex (ex Reheis) in ethanol was prepared. Independently, 50% solutions of oils Finsolv TN (C12-15 alkyl benzoate, ex Finetex) and Finsolv SUN (phenylethyl benzoate, ex Finetex) were also prepared. A sample of each of the oil solutions was added to an equal weight of the Reach 301 PGO solution to give the compositions indicated in Table 1. Addition of the Finsolv TN solution resulted in precipitation of the antiperspirant salt, whereas addition of the Finsolv SUN solution produced a clear, single phase composition.

**Table 1**

| **Component** | **Example A** | **Example 1** |
|---|---|---|
| Reach 301 PGO | 25 | 25 |
| Finsolv TN | 25 | -- |
| Finsolv SUN | -- | 25 |
| Ethanol | 50 | 50 |
| **Appearance**: | Opaque dispersion | Clear solution |

Examples according to the compositions indicated in Table 2 were prepared with a wide range of oils.

**Table 2**

| **Component** | **C1** | **C2** | **C3** | **C4** |
|---|---|---|---|---|
| Rehydrol II¹ | 15 | 15 | 15 | 15 |
| Ethanol | 47.8 | 52.8 | 40 | 45 |
| Oil² | 32.2 | 32.2 | 40 | 40 |
| Propylene glycol | 5 | -- | 5 | -- |

| | | | | |
|---|---|---|---|---|
| 1. ACH-PG antiperspirant complex, ex Reheis. 2. 28 different oils were formulated; details are given in Table 3. | | | | |

The initial stage in the preparation of compositions C1 to C4 was the mixing of the oil with ethanol (and propylene glycol in the cases of C1 and C3) to give compositions C1a to C4a, respectively (see Table 2a).

**Table 2a**

| **Component** | **C1a** | **C2a** | **C3a** | **C4a** |
|---|---|---|---|---|
| Ethanol | 46.9 | 54 | 35.8 | 42.9 |
| Oil² | 46 | 46 | 57.1 | 57.1 |
| Propylene glycol | 7.1 | -- | 7.1 | -- |

Compositions C1a to C4a were mixed with a 50% w/w solution of Rehydrol II to give compositions C1 to C4, respectively.

Table 3 gives details of the various oils formulated and of the stability of compositions C1 and C2 made using them. Instability was manifested by the initial oil solution (Cla or C2a) being unstable, in which case the full composition (C1 or C2) was not made ("n/m") or by the precipitation of the antiperspirant active out of solution ("no"), giving an opaque dispersion. Stability was assessed after storage of the composition for 18 hours at ambient temperature.

**Table 3**

| **Oil** | | | **Stability** | |
|---|---|---|---|---|
| **Trade name** | **INCI name** | **Supplier** | **C1** | **C2** |
| **Finsolv SUN** | Phenylethyl benzoate | Finetex | **Yes** | **Yes** |
| Finsolv TN | C12-15 alkyl benzoate | Finetex | No | Yes |
| **Finsolv PG22** | DPG dibenzoate | Finetex | **Yes** | **Yes** |
| Finsolv PPG15 | Stearyl ether Benzoate | Finetex | No | No |
| **Finsolv EB** | Octyl benzoate | Finetex | **Yes** | **Yes** |
| Finsolv BOD | Octyldodecyl Benzoate | Finetex | n/m | No |
| Finsolv BCO-115 | Castor oil benzoate | Finetex | n/m | n/m |
| Finsolv BCR-111 | Cetyl ricinoleate benzoate | Finetex | n/m | n/m |
| Finsolv BOHS-111 | Octyl hydroxystearate Benzoate | Finetex | n/m | n/m |
| **Finsolv PL-355** | Poloxamer 105 benzoate | Finetex | **Yes** | **Yes** |
| **Finsolv PL-62** | Poloxamer 182 dibenzoate | Finetex | **Yes** | **Yes** |
| Finsolv SB | Isostearyl benzoate | Finetex | n/m | n/m |
| **DG-1** | 1-methyl-2-phenoxy-ethyl benzoate | Degussa | **Yes** | **Yes** |
| Hallbrite TQ | Diethylhexyl 2,6-naphthalate | C.P.Hall | n/m | No |
| Hallbrite BHB | Butyloctyl salicylate | C.P.Hall | No | Yes |
| Estol 1514 | Isopropyl myristate | Unichema | No | Yes |
| Estol 1517 | Isopropyl palmitate | Unichema | No | No |
| Cetiol V | Decyl oleate | Cognis | n/m | No |
| Cetiol OE | Di-octyl ether | Cognis | No | No |
| Cetiol J600 | Liquid wax ester | Cognis | n/m | n/m |
| Cetiol CC | Di-n-octyl carbonate | Cognis | No | No |
| **Eutanol G** | Octyldodecanol | Cognis | **Yes** | **Yes** |
| **Prisorine 3515** | Isostearyl alcohol | Uniqema | **Yes** | **Yes** |
| Fluid AP | PPG-14 butyl ether | Union Carbide | No | No |
| **Promyristyl PM3** | PPG3 myristyl ether | Croda | **Yes** | **Yes** |
| **DC245** | Cyclomethicone | Dow Corning | **Yes** | **Yes** |
| DC200 (5 cst) | Dimethicone | Dow Corning | n/m | n/m |
| Sirius M70 | Mineral oil | Silkolene | n/m | n/m |

There were 10 "successful" oils in the above screening test (entries emboldened), each allowing both C1 and C2 to be prepared as a stable compositions. Each of these ten oils was formulated into compositions C3 and C4, the stabilities of which were assessed over 18 hours at ambient temperature. The results are shown in Table 4.

**Table 4**

| **Oil** | | **Composition stability** | |
|---|---|---|---|
| **Designation** | **Trade name** | **C3** | **C4** |
| **2** | Finsolv SUN | Yes | Yes |
| **3** | Finsolv PG22 | Yes | Yes |
| **4** | (Finsolv EB | Yes | Yes |
| | Finsolv PL-355 | No | Yes |
| | Finsolv PL-62 | No | No |
| **5** | DG-1 | Yes | Yes |
| | Eutanol G | No* | Yes |
| **6** | Pristorine 3515 | Yes | Yes |
| | Promyristyl PM3 | No* | Yes |
| | DC 245 | No | No |

| | | | |
|---|---|---|---|
| * The AP active did not immediately precipitate out of these compositions - they were on the borderline of stability. | | | |

From Table 4, it can be seen that at the higher oil levels required by C3 and C4, only 5 of the oils allowed both C3 and C4 to be prepared as stable compositions. A further three oils (Finsolv PL-355, Eutanol G, and Promyristyl PM3) allowed C4 to be prepared as a stable composition and two of these oils (Eutanol G and Promyristyl PM3) also allowed C3 to be prepared with 'borderline' stability.

The oils allowing both C3 and C4 to be prepared as stable compositions, designated 2 to 6 in Table 4 and Table 5, were formulated into compositions C1 to C4 and the stability of these compositions was assessed by storing them for 3 months at 4°C and at 45°C. The results are shown in Table 5. The samples were examined after 24 hours, after 2 weeks, after 2 months, and after 3 months. The length of time for which stability is indicated means that the composition had phase separated at the next examination, with the exception of the 3 month figure (the test terminating at this time).

**Table 5**

| **Oil** | **Composition stability** (in months, unless otherwise indicated: h = hours, w = weeks, 0 = less than 24 h) | | | | | | | |
|---|---|---|---|---|---|---|---|---|
| | **At 4°C** | | | | **At 45°C** | | | |
| | **C1** | **C2** | **C3** | **C4** | **C1** | **C2** | **C3** | **C4** |
| **2** | 3 | 3 | 24h* | 3 | 2 | 2 | 2w | 2w |
| **3** | 3 | 3 | 3 | 3 | 2 | 3* | 2w | 2 |
| **4** | 3 | 3 | 3 | 3 | 2 | 2 | 24h | 24h |
| **5** | 3 | 3 | 0 | 3 | 2 | 2 | 2w | 2w |
| **6** | 3 | 3 | 3 | 3 | 3* | 3 | 2 | 3 |

| | | | | | | | | |
|---|---|---|---|---|---|---|---|---|
| * Appeared cloudy, but without full precipitation, at this assessment time. | | | | | | | | |

The results in Table 5 illustrate that all of the selected oils (2-6) allow the formulation of C1, C2, and C4 compositions having excellent low temperature stability. Oils 3, 4, and 6 (Finsolv PG22, Finsolv EB, and Prisorine 3515) also allowed the formulation of a C3 composition having excellent low temperature stability.

The results in Table 5 show that high temperature stability proved harder to achieve. Oil 6 (Prisorine 3515) was the most successful; it allowed the formulation of compositions lacking propylene glycol (C2 and C4) having excellent high temperature stability and the formulation of compositions having propylene glycol (C1 and C3) having stability at 45°C for at least two months. Oil 4 (Finsolv EB) was the least successful, compositions C3 and C4 becoming opaque dispersions within two weeks. Of the other three oils, the high temperature stability of the compositions was identical for oil 2 (Finsolv SUN) and oil 5 (DG-1) and marginally superior for oil 3 (Finsolv PG22).

Examples 7, 8 and 9 indicated in Table 6 have good stability at temperatures from 4°C to 50°C. This is an indication of the good stability of compositions according to the invention comprising ISA and volatile silicone oil, even in the presence of perfume, and of compositions according to the invention comprising ISA, a dibenzoate ester of a C6 dihydric alcohol, and a perfume.

**Table 6**

| **Component** | **Example 7** | **Example 8** | **Example 9** |
|---|---|---|---|
| Rehydrol II | 15 | 15 | 15 |
| Ethanol | 40 | 35 | 30 |
| DC 245 | -- | 10 | 10 |
| Prisorine 3515 | 32 | 40 | 37 |
| Finsolv PG-22 | 10 | -- | -- |
| Perfume | 3 | -- | 3 |

'Hotroom tests' have been performed using the three Examples indicated in Table 7. The test involved the pump spray application of 0.31g of the each composition to the axillae of approximately 30 female panellists and the "SWR" figures shown at the foot of the table are the average sweat weight reductions found (compared with no product application). A statistically significant reduction in perspiration was obtained on use of each of the compositions.

**Table 7**

| **Component** | **Example 10** | **Example 11** | **Example 12** |
|---|---|---|---|
| Reach 310 PG | 15 | -- | -- |
| Rehydrol II | -- | 15 | 15 |
| Ethanol | 40.2 | 30 | 35 |
| DC 245 | -- | 5 | 10 |
| Prisorine 3515 | -- | 40 | 35 |
| Finsolv SUN | 42 | -- | -- |
| Finsolv TN | -- | 5 | -- |
| Propylene glycol | 2.8 | 5 | 5 |
| **SWR (%):** | **34** | **25** | **30** |

## Claims

1. An anhydrous antiperspirant composition comprising less than 10% by weight of water; from 2 to 25% by weight of solubilised aluminium-containing antiperspirant active; from 20 to 50% by weight of ethanol and from 20 to 55% by weight of one or more solubiliser oils selected from the group consisting of:
(i). a branched-chain fatty alcohol having from 16 to 24 carbon atoms;
(ii). a benzoate ester of a short chain (C2-C6) alcohol substituted with a phenyl, phenoxy or benzoyloxy group;
(iii). a benzoate ester of a straight-chain alcohol of from 6 to 8 carbon atoms; and
(iv). a propoxylated linear fatty alcohol having a degree of propoxylation of from 2 to 4 and a fatty alcohol chain length of from 10 to 18 carbon atoms;
wherein the ratio of antiperspirant active to ethanol and of antiperspirant active to the solubiliser oil is from 1:10 to 1:1 by weight and the ratio of ethanol to oil is from 1:2 to 2:1 by weight.

2. An antiperspirant composition according to claim 1, that is a liquid composition.

3. An antiperspirant composition according to claim 1 or claim 2, that is a solution.

4. An antiperspirant composition according to any of the preceding claims, that is a spray composition that does not comprise a volatile propellant.

5. An antiperspirant composition according to any of the preceding claims, wherein the AP active is soluble in ethanol.

6. An antiperspirant composition according to claim 5, wherein the AP active is an aluminium chlorohydrate complex with propylene glycol.

7. An antiperspirant composition according to any of the preceding claims, wherein the solubiliser oil is selected from the group consisting of:
(i). a branched-chain fatty alcohol having from 16 to 24 carbon atoms; and
(ii). a benzoate ester of a short chain (C2-C6) alcohol substituted with a phenyl, phenoxy or benzoyloxy group.

8. An antiperspirant composition according to claim 7, wherein the solubiliser oil is a branched-chain fatty alcohol having from 16 to 24 carbon atoms.

9. An antiperspirant composition according to claim 8, wherein the solubiliser oil is isostearyl alcohol.

10. An antiperspirant composition according to any of claims 1 to 7, wherein the solubiliser oil is a benzoate ester of a short chain (C2-C6) alcohol substituted with a phenyl, phenoxy or benzoyloxy group.

11. An antiperspirant composition according to claim 10, wherein the solubiliser oil is a dibenzoate ester of a short chain (C2-C6) dihydric alcohol.

12. An antiperspirant composition according to claim 11, comprising an additional solubiliser oil that is a branched-chain fatty alcohol having from 16 to 24 carbon atoms.

13. An antiperspirant composition according to any of the preceding claims, wherein the ratio of antiperspirant active to ethanol and of antiperspirant active to the solubiliser oil is from 1:5 to 1:1 by weight.

14. An antiperspirant composition according to any of the preceding claims, wherein the ratio of ethanol to oil is from 2:3 to 3:2 by weight.

15. An antiperspirant composition according to any of the preceding claims, comprising a silicone oil.

16. An antiperspirant composition according to claim 15, comprising cylcopentasiloxane.

17. An antiperspirant composition according to any of the preceding claims, comprising a short chain (C2-C6) polyhydric alcohol.

18. An antiperspirant composition according to claim 17, comprising propylene glycol.

19. A method of manufacture of a composition according claim 1, said method comprising the preparation of a solution of the solubiliser oil in ethanol, the independent preparation of a solution of the AP active in ethanol, and then the mixing of the two solutions.

20. A cosmetic method of reducing perspiration, comprising the application to the surface of the human body of a composition according to any of claims 1 to 18.

21. A cosmetic method according to claim 20, comprising the application of a composition according to claim 4 using a spray dispenser.

22. A product comprising an antiperspirant composition according to any of the preceding claims and a spray dispenser, roll-on dispenser, or porous head dispenser.

## Patentansprüche

1. Wasserfreie Antiperspirant-Zusammensetzung, umfassend weniger als 10 Gew.-% Wasser, 2 bis 25 Gew.-% löslich gemachte, Aluminium enthaltende Antiperspirant-Aktivsubstanz, 20 bis 50 Gew.-% Ethanol und 20 bis 55 Gew.-% eines oder mehrerer Lösungsvermittleröle, ausgewählt aus der Gruppe bestehend aus:
(i) einem verzweigtkettigen Fettalkohol mit 16 bis 24 Kohlenstoffatomen,
(ii) einem Benzoatester eines kurzkettigen (C2-C6) Alkohols, substituiert mit einer Phenyl-, Phenoxy- oder Benzoyloxy-Gruppe,
(iii) einem Benzoatester eines geradkettigen Alkohols mit 6 bis 8 Kohlenstoffatomen und
(iv) einem propoxylierten linearen Fettalkohol mit einem Propoxylierungsgrad von 2 bis 4 und einer Fettalkoholkettenlänge von 10 bis 18 Kohlenstoffatomen,
worin das Gewichtsverhältnis der Antiperspirant-Aktivsubstanz zum Ethanol und der Antiperspirant-Aktivsubstanz zu dem Lösungsvermittleröl 1:10 bis 1:1 und das Gewichtsverhältnis des Ethanols zu dem Öl 1:2 bis 2:1 beträgt.

2. Antiperspirant-Zusammensetzung nach Anspruch 1, welche eine flüssige Zusammensetzung ist.

3. Antiperspirant-Zusammensetzung nach Anspruch 1 oder 2, welche eine Lösung ist.

4. Antiperspirant-Zusammensetzung nach einem der vorhergehenden Ansprüche, welche eine Sprüh-Zusammensetzung ist, die kein flüchtiges Treibmittel enthält.

5. Antiperspirant-Zusammensetzung nach einem der vorhergehenden Ansprüche, worin die AP-Aktivsubstanz in Ethanol löslich ist.

6. Antiperspirant-Zusammensetzung nach Anspruch 5, worin die AP-Aktivsubstanz ein Aluminiumchlorhydrat-Komplex mit Propylenglykol ist.

7. Antiperspirant-Zusammensetzung nach einem der vorhergehenden Ansprüche, worin das Lösungsvermittleröl aus der Gruppe ausgewählt ist, die besteht aus
(i) einem verzweigtkettigen Fettalkohol mit 16 bis 24 Kohlenstoffatomen und
(ii) einem Benzoatester eines kurzkettigen (C2-C6) Alkohols, der mit einer Phenyl-, Phenoxy- oder Benzoyloxy-Gruppe substituiert ist.

8. Antiperspirant-Zusammensetzung nach Anspruch 7, worin das Lösungsvermittleröl ein verzweigtkettiger Fettalkohol mit 16 bis 24 Kohlenstoffatomen ist.

9. Antiperspirant-Zusammensetzung nach Anspruch 8, worin das Lösungsvermittleröl Isostearylalkohol ist.

10. Antiperspirant-Zusammensetzung nach einem der Ansprüche 1 bis 7, worin das Lösungsvermittleröl ein Benzoatester eines kurzkettigen (C2-C6) Alkohols ist, der mit einer Phenyl-, Phenoxy- oder Benzoyloxy-Gruppe substituiert ist.

11. Antiperspirant-Zusammensetzung nach Anspruch 10, worin das Lösungsvermittleröl ein Dibenzoatester eines kurzkettigen (C2-C6) zweiwertigen Alkohols ist.

12. Antiperspirant-Zusammensetzung nach Anspruch 11, umfassend ein zusätzliches Lösungsvermittleröl, das ein verzweigtkettiger Fettalkohol mit 16 bis 24 Kohlenstoffatomen ist.

13. Antiperspirant-Zusammensetzung nach einem der vorhergehenden Ansprüche, worin das Gewichtsverhältnis der Antiperspirant-Aktivsubstanz zum Ethanol und der Antiperspirant-Aktivsubstanz zu dem Lösungsvermittleröl 1:5 bis 1:1 berägt.

14. Antiperspirant-Zusammensetzung nach einem der vorhergehenden Ansprüche, worin das Gewichtsverhältnis von Ethanol zu Öl 2:3 bis 3:2 beträgt.

15. Antiperspirant-Zusammensetzung nach einem der vorhergehenden Ansprüche, umfassend ein Silikonöl.

16. Antiperspirant-Zusammensetzung nach Anspruch 15, umfassend Cyclopentasiloxan.

17. Antiperspirant-Zusammensetzung nach einem der vorhergehenden Ansprüche, umfassend einen kurzkettigen (C2-C6) mehrwertigen Alkohol.

18. Antiperspirant-Zusammensetzung nach Anspruch 17, umfassend Propylenglykol.

19. Verfahren zur Herstellung einer Zusammensetzung gemäß Anspruch 1, wobei das Verfahren die Herstellung einer Lösung eines Lösungsvermittleröls in Ethanol, die unabhängige Herstellung einer Lösung der AP-Aktivsubstanz in Ethanol und anschließendes Vermischen der zwei Lösungen umfaßt.

20. Kosmetisches Verfahren zur Verminderung der Schweißbildung, umfassend den Auftrag einer Zusammensetzung nach irgendeinem der Ansprüche 1 bis 18 auf die Oberfläche des menschlichen Körpers.

21. Kosmetisches Verfahren nach Anspruch 20, umfassend den Auftrag einer Zusammensetzung gemäß Anspruch 4 unter Verwendung eines Zerstäubers.

22. Produkt, umfassend eine Antiperspirant-Zusammensetzung nach irgendeinem der vorhergehenden Ansprüche und einen Zerstäuber, ein Rollauftragsgerät oder ein Auftragsgerät mit porösem Kopf.

## Revendications

1. Composition anti-transpiration anhydre comprenant moins de 10 % en poids d'eau ; de 2 à 25 % en poids d'un agent anti-transpiration actif contenant de l'aluminium solubilisé ; de 20 à 50 % en poids d'éthanol et de 20 à 55 % en poids d'une ou de plusieurs huiles solubilisantes choisies dans le groupe constitué par :
(i) un alcool gras à chaîne ramifiée ayant de 16 à 24 atomes de carbone ;
(ii) un ester de benzoate d'un alcool à chaîne courte (C2-C6) substitué par un groupe phényle, phénoxy ou benzoyloxy ;
(iii) un ester de benzoate d'un alcool à chaîne droite ayant de 6 à 8 atomes de carbone ; et
(iv) un alcool gras linéaire propoxylé ayant un degré de propoxylation de 2 à 4 et une longueur de chaîne d'alcool gras de 10 à 18 atomes de carbone ;
dans laquelle le rapport de l'agent anti-transpiration actif à l'éthanol et de l'agent anti-transpiration actif à l'huile solubilisante est de 1:10 à 1:1 en poids et le rapport de l'éthanol à l'huile est de 1:2 à 2:1 en poids.

2. Composition anti-transpiration selon la revendication 1, qui est une composition liquide.

3. Composition anti-transpiration selon la revendication 1 ou la revendication 2, qui est une solution.

4. Composition anti-transpiration selon l'une quelconque des revendications précédentes, qui est une composition en spray qui ne comprend pas de gaz propulseur volatil.

5. Composition anti-transpiration selon l'une quelconque des revendications précédentes, dans laquelle l'agent anti-transpiration actif est soluble dans l'éthanol.

6. Composition anti-transpiration selon la revendication 5, dans laquelle l'agent anti-transpiration actif est un complexe d'hydrochlorate d'aluminium avec le propylène glycol.

7. Composition anti-transpiration selon l'une quelconque des revendications précédentes, dans laquelle l'huile solubilisante est choisie dans le groupe constitué par :
(i) un alcool gras à chaîne ramifiée ayant de 16 à 24 atomes de carbone ; et
(ii) un ester de benzoate d'un alcool à chaîne courte (C2-C6) substitué par un groupe phényle, phénoxy ou benzoyloxy.

8. Composition anti-transpiration selon la revendication 7, dans laquelle l'huile solubilisante est un alcool gras à chaîne ramifiée ayant de 16 à 24 atomes de carbone.

9. Composition anti-transpiration selon la revendication 8, dans laquelle l'huile solubilisante est l'alcool isostéarylique.

10. Composition anti-transpiration selon l'une quelconque des revendications 1 à 7, dans laquelle l'huile solubilisante est un ester de benzoate d'un alcool à chaîne courte (C2-C6) substitué par un groupe phényle, phénoxy ou benzoyloxy.

11. Composition anti-transpiration selon la revendication 10, dans laquelle l'huile solubilisante est un ester de dibenzoate d'un dialcool à chaîne courte (C2-C6).

12. Composition anti-transpiration selon la revendication 11 comprenant une huile solubilisante supplémentaire qui est un alcool gras à chaîne ramifiée ayant de 16 à 24 atomes de carbone.

13. Composition anti-transpiration selon l'une quelconque des revendications précédentes, dans laquelle le rapport de l'agent anti-transpiration actif à l'éthanol et de l'agent anti-transpiration actif à l'huile solubilisante est de 1:5 à 1:1 en poids.

14. Composition anti-transpiration selon l'une quelconque des revendications précédentes, dans laquelle le rapport de l'éthanol à l'huile est de 2:3 à 3:2 en poids.

15. Composition anti-transpiration selon l'une quelconque des revendications précédentes comprenant une huile silicone.

16. Composition anti-transpiration selon la revendication 15 comprenant un cyclopentasiloxane.

17. Composition anti-transpiration selon l'une quelconque des revendications précédentes comprenant un polyalcool à chaîne courte (C2-C6).

18. Composition anti-transpiration selon la revendication 17 comprenant un polypropylène glycol.

19. Procédé de fabrication d'une composition selon la revendication 1, ledit procédé comprenant la préparation d'une solution de l'huile solubilisante dans l'éthanol, la préparation indépendante d'une solution de l'agent anti-transpiration actif dans l'éthanol, puis le mélange des deux solutions.

20. Procédé cosmétique permettant de réduire la transpiration, comprenant l'application à la surface du corps humain d'une composition selon l'une quelconque des revendications 1 à 18.

21. Procédé cosmétique selon la revendication 20, comprenant l'application d'une composition selon la revendication 4 au moyen d'un distributeur de type spray.

22. Produit comprenant une composition anti-transpiration selon l'une quelconque des revendications précédentes et un distributeur de type spray, un distributeur de type à bille, ou un distributeur à tête poreuse.
